# EUROPEAN PATENT SPECIFICATION

(11) **EP 4 262 935 B1**
(45) Date of publication and mention of the grant of the patent: **06.05.2026**
(21) Application number: 21819890.1
(22) Date of filing: 03.12.2021
(51) Int. Cl.: A61M 5/32, A61M 5/34

(54) **MEDICAMENT DELIVERY MEMBER HOUSING ASSEMBLY**
GEHÄUSEANORDNUNG FÜR MEDIKAMENTENVERABREICHUNGSELEMENT
ENSEMBLE BOÎTIER D'ÉLÉMENT D'ADMINISTRATION DE MÉDICAMENT

(30) Priority: 16.12.2020 EP 20214486
(43) Date of publication of application: 25.10.2023
(73) Proprietor: SHL Medical AG, 6302 Zug (CH)
(72) Inventor: YIN, Ming-Ting, Taoyuan City, 338 (TW)
(86) International application number: PCT/EP2021/084186
(87) International publication number: WO 2022/128533

(56) References cited:
- US-A1- 2011 125 100
- US-A1- 2015 273 161

## Description

### TECHNICAL FIELD

The invention concerns medicament delivery member housing assemblies, and particularly medicament delivery member housing assemblies with shields protecting the medicament delivery member.

### BACKGROUND

In cartridge-based medicament delivery devices such as autoinjectors, it is important that the medicament delivery member (e.g. a needle) is kept safe before use, both to avoid needle stick injuries and also to maintain sterility. In addition, cartridge-based medicament delivery devices are typically provided with the medicament delivery member separated from the medicament container, which means that the user typically has to somehow attach the medicament delivery member to the medicament container before carrying out an injection. Some examples can be found in US2011/125100 and US2015/273161. This adds extra steps and therefore extra complexity to device usage, which is undesirable in terms of ease of use. Considering this, the applicant has appreciated that improvements can be made in comparison to existing designs.

### SUMMARY

The invention is defined by the appended claims, to which reference should now be made.

In the present disclosure, when the term "distal direction" is used, this refers to the direction pointing away from the dose delivery site during use of the medicament delivery device. When the term "distal part/end" is used, this refers to the part/end of the delivery device, or the parts/ends of the members thereof, which under use of the medicament delivery device is/are located furthest away from the dose delivery site. Correspondingly, when the term "proximal direction" is used, this refers to the direction pointing towards the dose delivery site during use of the medicament delivery device. When the term "proximal part/end" is used, this refers to the part/end of the delivery device, or the parts/ends of the members thereof, which under use of the medicament delivery device is/are located closest to the dose delivery site.

Further, the terms "longitudinal", "longitudinally", "axially" and "axial" refer to a direction extending from the proximal end to the distal end and along the device or components thereof, typically in the direction of the longest extension of the device and/or component.

Similarly, the terms "transverse", "transversal" and "transversally" refer to a direction generally perpendicular to the longitudinal direction.

An aspect of the invention concerns a medicament delivery member housing assembly extending along an axis in a longitudinal direction from a proximal end to a distal end, the medicament delivery member housing assembly comprising: a housing comprising a body and a flexible arm, the flexible arm extending in the longitudinal direction from a proximal end moveable relative to the body to a distal end attached to the body, wherein the flexible arm comprises a first surface facing towards the proximal end of the medicament delivery member housing assembly and a second surface facing in a radial direction relative to the axis; a medicament delivery member; a proximal shield extending around a first end of the medicament delivery member, the proximal shield comprising a surface facing towards the distal end of the medicament delivery member housing assembly to interact with the first surface of the housing during use; and a distal shield extending around a distal end of the medicament delivery member, the distal shield being slidable in the longitudinal direction relative to the proximal shield, the distal shield comprising a surface facing in the radial direction to interact with the second surface of the housing during use.

This design can provide a built-in needle-housing assembly for cartridge-based autoinjectors. This design can first connect the distal end of the needle to a cartridge and penetrate the proximal end of the proximal shield and subsequently an injection site with a simple linear compressive motion. As such, it can be implemented into a device activation mechanism (e.g. a needle cover compression), and can thereby avoid the need for any additional user preparation steps.

Preferably, the medicament delivery member housing assembly comprises a needle attached to the housing. Preferably, the proximal end of the medicament delivery member housing assembly is the end of the medicament delivery member housing assembly closest to the site of delivery of a medicament during use. With the housing assembly this way around, a medicament container can be penetrated before a medicament delivery member extends out of the proximal end of the housing assembly.

Preferably, the medicament delivery member housing assembly is configured to move from an initial extended position in which the needle is fully enclosed by the medicament delivery member housing assembly to a final compressed position where the needle extends out of the proximal end of the proximal shield and where the needle extends out of the distal end of the distal shield.

Preferably, the medicament delivery member housing assembly is configured to move from the initial extended position to an intermediate partially-extended position and then to a final compressed position, wherein in the initial extended position, the first surface of the flexible arm and the surface of the proximal shield are aligned in the longitudinal direction, wherein in the intermediate partially-extended position, the first surface of the flexible arm and the surface of the proximal shield are offset from one another in the longitudinal direction and adjacent to one another in the radial direction, and the second surface of the flexible arm is adjacent to the surface of the distal shield, wherein in the final compressed position, the surface of the proximal shield is further from the proximal end of the proximal shield than the first surface of the flexible arm is from the proximal end of the proximal shield.

Preferably, the second surface of the flexible arm faces away from the axis in the radial direction and the surface of the distal shield faces towards the axis in the radial direction. Preferably, the proximal end of the proximal shield comprises a septum which the medicament delivery member can penetrate. Preferably, the distal end of the distal shield comprises a septum which the medicament delivery member can penetrate. This can allow use of different materials for the area being penetrated and for other parts of the shields.

Preferably, the first surface of the flexible arm and the surface of the proximal shield are spaced apart in the longitudinal direction. This can reduce the force needed to initiate movement of the proximal shield relative to the distal shield.

Preferably, the medicament delivery member housing assembly comprises a seal arranged between the proximal shield and the distal shield. Preferably, the seal is attached to only one of the proximal shield and the distal shield. Preferably, the one of the proximal shield and the distal shield that the seal is not attached to comprises a recess, and the seal extends into the recess.

Preferably, the one of the proximal shield and the distal shield that the seal is not attached to comprises a protrusion extending in the radial direction, and the seal abuts the protrusion. This can allow the seal to be spaced apart from the outer surface of the one of the proximal shield and the distal shield that the seal is not attached to, which can reduce friction during use (as the seal does not rub against the one of the proximal shield and the distal shield that the seal is not attached to as the proximal shield moves relative to the distal shield)
Preferably, the proximal end of the flexible arm is moveable relative to the body in a radial direction relative to the axis. Preferably, the proximal shield is tubular and the distal shield is tubular, and the diameter of the distal shield is wider than the diameter of the proximal shield, in the direction perpendicular to the longitudinal axis. Preferably, the housing is attached to the distal shield by a snap fit and/or the distal shield is attached to the proximal shield by a snap fit. Preferably, the distal shield has a greater radius when measured perpendicular to the axis than the proximal shield.

Another aspect concerns a cassette comprising a medicament delivery member housing assembly as described above. Preferably, the medicament delivery member housing assembly is attached to the cassette by a snap-fit.

Another aspect concerns an autoinjector comprising a medicament delivery member housing assembly as described above or comprising a cassette as described above.

Another aspect concerns a method of operating a medicament delivery member housing assembly, comprising the steps of: pushing a proximal shield of the medicament delivery member housing assembly in the distal direction relative to a distal shield of the medicament delivery member housing assembly, so that a body of the medicament delivery member housing assembly is also pushed in the distal direction relative to the distal shield by the interaction between a surface of the proximal shield and a first surface of the body until a distal end of a medicament delivery member of the body penetrates a distal end of the distal shield, then pushing the proximal shield further in the distal direction relative to the distal shield and also relative to the body, until a proximal end of the medicament delivery member penetrates a proximal end of the proximal shield.

Generally, all terms used in the claims are to be interpreted according to their ordinary meaning in the technical field, unless explicitly defined otherwise herein. All references to a/an/the element, apparatus, member, component, means, etc. are to be interpreted openly as referring to at least one instance of the element, apparatus, member component, means, etc., unless explicitly stated otherwise.

### BRIEF DESCRIPTION OF THE DRAWINGS

Embodiments of the invention will now be described by way of example only and with reference to the accompanying drawings, in which:
Figure 1 shows a perspective view of a needle-housing assembly.
Figure 2 shows a perspective view of the parts of the needle-housing assembly of Figure 1.
Figure 3 shows a cross-section view of the needle-housing assembly of Figure 1.
Figure 4 shows a cross-section view of the needle-housing assembly of Figure 1 at a 90-degree angle in the circumferential direction relative to the cross-section view in Figure 3.
Figure 5 shows a perspective view of the housing of Figure 1.
Figure 6 shows a perspective view of the proximal shield of Figure 1.
Figure 7 shows a perspective view of the distal shield of Figure 1.
Figure 8 shows a partially transparent perspective view of another needle-housing assembly before use.
Figure 9 shows the needle-housing assembly of Figure 8 partially compressed.
Figure 10 shows the needle-housing assembly of Figure 8 fully compressed.
Figure 11 shows a cross-section view of the compressed needle-housing assembly in Figure 10.
Figure 12 shows a perspective view of a cassette comprising a needle-housing assembly according to Figure 1.
Figure 13 shows a cross-section view of part of the cassette of Figure 12.
Figure 14 shows a cross-section view of part of another needle-housing assembly.
Figure 15 shows a close-up view of part of Figure 14.
Figure 16 shows the same view as Figure 15, but during use.
Figure 17 shows a cross-section view of how the distal shield could attach to a cassette.
Figure 18 shows a cross-section view of the embodiment of Figure 17 at a 90-degree angle in the circumferential direction relative to the cross-section view in Figure 17.

### DETAILED DESCRIPTION

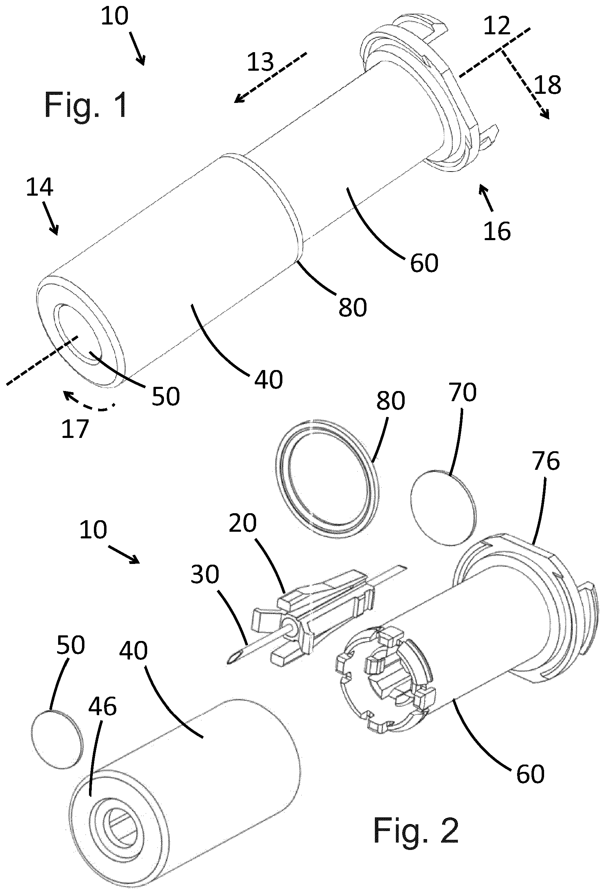

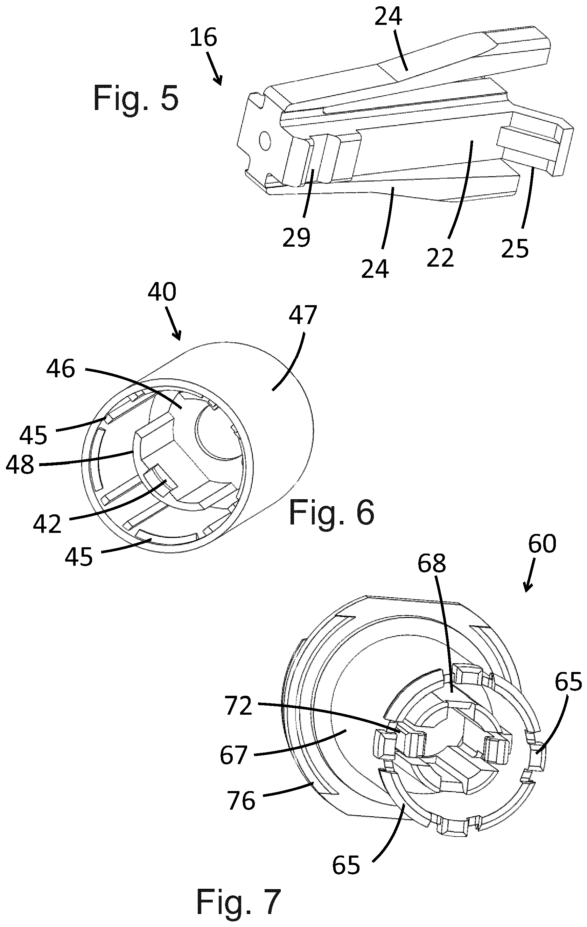

Figure 1 shows a needle-housing assembly 10 extending along an axis 12 in a longitudinal direction 13 from a proximal end 14 to a distal end 16, the needle-housing assembly 10 comprising a housing (see Figure 2), a proximal shield 40 (first shield) and a distal shield 60 (second shield). Figure 2 shows the individual parts of the needle-housing assembly 10 shown in Figure 1, including a septum 50 (seal) of the proximal shield 40, the housing 20, a needle 30 attached to the housing 20, a seal 80 between the proximal shield 40 and the distal shield 60, and a septum 70 (seal) of the distal shield 60. Figures 5 to 7 show further detail of the housing 20, the proximal shield 40 and the distal shield 60.

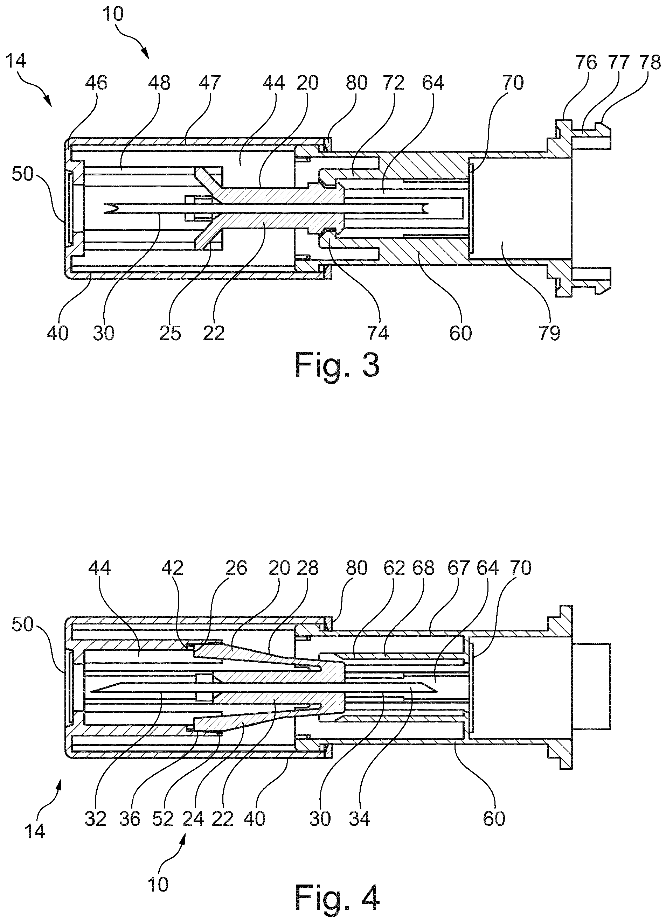

Figures 3 and 4 show cross sections of the needle-housing assembly 10, with the two cross sections at 90 degrees relative to each other to show some of the circumferential variability in structure. The proximal shield 40, which is shown in more detail in Figure 6, comprises a proximal wall 46, an outer tubular portion 47 attached to the proximal wall 46 and two inner arms 48 attached to the proximal wall 46. The inner arms 48 are inside the outer tubular portion 47. The inner arms 48 extend in the distal direction from the proximal wall 46. The inner arms 48 each comprise a surface 42. The surface 42 faces towards a first surface 26 of the housing 20, which in this case means that the surface 42 faces in the distal direction.

The housing 20, which is shown in more detail in Figure 5, comprises a body 22 and a flexible arm 24. The needle 30 is attached to the body. The proximal end 32 of the needle extends from the proximal end of the housing, and the distal end 34 of the needle extends from the distal end of the housing. The flexible arm 24 extends from a distal end to a proximal end in the longitudinal direction. The distal end of the flexible arm 24 is attached to the body 22, and the proximal end of the flexible arm 24 is moveable relative to the body 22 in the radial direction 18 relative to the axis 12. The proximal end of the flexible arm 24 comprises a first surface facing towards the proximal end of the medicament delivery member housing assembly, and in particular towards the surface 42.

The housing 20 also comprises an optional pair of flanges 25 at the proximal end of the housing. When the needle-housing assembly is assembled, the flanges 25 do not overlap with the arms 48 of the optional shield in the circumferential direction 17, but the flanges 25 do overlap with the arms 48 in the axial direction. This can help with assembly, and can also help ensure that the body and the proximal shield cannot rotate relative to each other (which could otherwise result in the surface of the proximal shield and the first surface of the body moving out of alignment with one another).

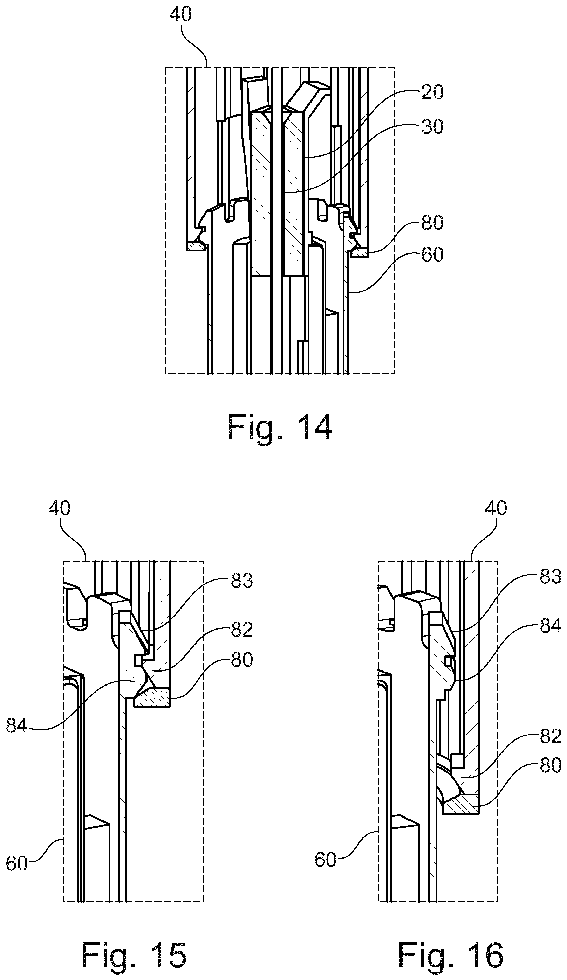

As can be seen from Figure 4 in particular, the housing interacts with the proximal shield. The proximal shield 40 and the distal shield 60 are slidably arranged relative to one another, with an optional seal 80 to seal the gap between them. The seal 80 is attached to the proximal shield 40 in this example but may be attached to either the proximal shield 40 or the distal shield 60. The proximal shield and the distal shield may be attached to one another in various ways which will be described below, for example by a snap fit as shown in Figure 14.

The distal shield 60, which is shown in more detail in Figure 7, comprises an outer tubular portion 67, an inner tubular portion 68 inside the outer tubular portion 67, arms 72 at the proximal end of the distal shield 60 and an optional flange 76 at the distal end of the distal shield 60. The arms 72 are flexible and extend from a distal end attached to the inner tubular portion 68 to a proximal end. Each arm 72 comprises a protrusion 74 at the proximal end of the arm 72, with the protrusion shaped to engage with a recess 29 of the housing in a snap fit as described in more detail below, although other snap fit structures or other attachment arrangements could be used instead.

As can be seen from Figure 3 in particular, the housing interacts with the distal shield with a connection that allows the body to move in the distal direction relative to the housing and restricts movement of the body in the proximal direction relative to the housing. The distal shield interacts with the housing 20 (specifically the body 22 of the housing 20 in this example) through the connection; specifically by using protrusions 74 on arms 72 of the distal shield 60 and the recess 29 on the housing 20. Interaction between a proximally facing surface of the recess 29 and a distally facing surface of the corresponding protrusion 74 restricts movement of the body in the proximal direction relative to the distal shield. The arms 72 of the distal shield 60 are flexible. Interaction between a distally facing surface of the recess 29 and a corresponding proximally facing surface of the protrusion 74, which face each other and which are angled relative to the axis, holds the housing and the distal shield in place relative to each other but allows the housing to move in the distal direction relative to the distal shield when a compressive force is applied to the needle-housing assembly (i.e. the compressive force overcomes the snap fit). The connection between the body and the distal housing as described above is just one example of how the two parts could be attached and held in position relative to one another. A flexible ring could instead be used rather than flexible arms, for example, or other features of the body and the distal housing could be used to hold the body and the distal housing in position relative to one another. A friction lock could also be used. Similarly, the distal shield and the proximal shield can optionally be supported relative to one another in various ways, for example by a number of ribs 45 on the proximal shield interacting with a number of protrusions 65 on the distal shield as can be seen in particular in Figures 6 and 7, to restrict movement in the longitudinal direction relative to one another and movement rotationally relative to one another. Further discussion of how the proximal shield and the distal shield can be attached to one another is outlined below.

As can be seen in Figure 7 in particular, the distal shield optionally comprises an attachment to attach the needle-housing mechanism to another part of a medicament delivery device; for example, the distal shield 60 optionally comprises a distal arm 77, which may be flexible, and which may comprise a protrusion 78.

Optionally, the distal arm 77 extends from a flange 76 at the distal end of the distal shield 60. The protrusion 78 may alternatively be arranged directly on the distal shield 60, in which case the distal arm 77 and the flange 76 may be optional.

The needle-housing assembly includes two cavities 44, 64 that generally fully enclose the needle. The first cavity (the cavity 44 of the proximal shield 40) is primarily formed by the proximal shield 40 (specifically the proximal wall 46 and the outer tubular portion 47), with enclosure of the cavity being completed by the proximal end of the distal shield 60 and the body 22, along with the optional seal 80 between the proximal shield and the distal shield. The second cavity (the cavity 64 of the distal shield 60) is primarily formed by the distal shield 60 (specifically the inner tubular portion of the distal shield), with enclosure of the cavity being completed by the body 22. The two cavities 44, 64 are not necessarily isolated from one another, as can be seen in Figure 4 for example, and indeed a connection enabling fluid to flow from the proximal cavity 44 to the distal cavity 64 can be beneficial as it can reduce pressure variation within the cavities 44, 64 during compression of the needle-housing assembly.

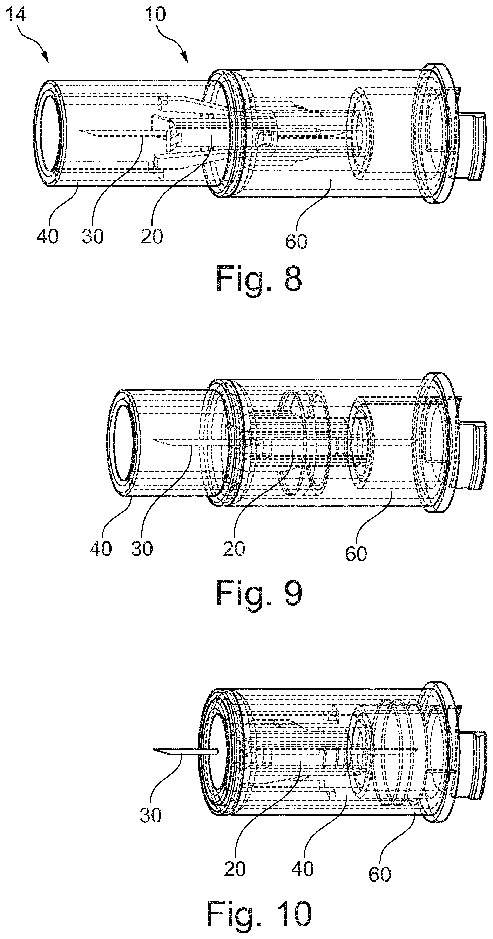

Figures 8 to 10 show another example of a needle-housing assembly. In this example, the distal shield has a greater radius than the proximal shield (specifically, the outer tubular portion 67 of the distal shield has a greater radius than the outer tubular portion 47 of the proximal shield), in contrast to the example shown in Figures 1 to 7, where the distal shield has a smaller radius than the proximal shield (specifically, the outer tubular portion 67 of the distal shield has a smaller radius than the outer tubular portion 47 of the proximal shield). This means that the proximal shield of the example in Figures 8 to 10 ends up inside the distal shield during use rather than vice versa.

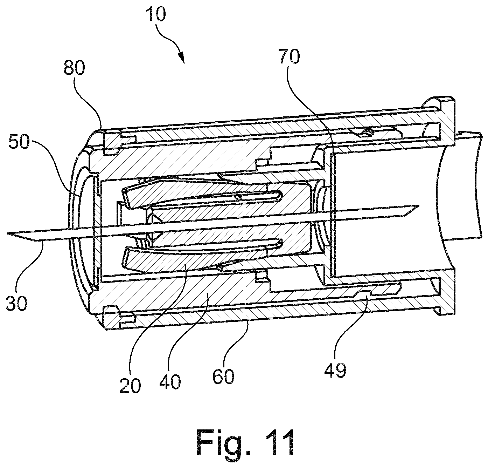

Figures 8 to 11 will now be used to describe how the needle-housing assembly 10 works. The same functionality is also provided by the example given in Figures 1 to 7. Figure 8 shows the initial position after assembly and before the needle-housing assembly is used, in which the needle-housing assembly is in an extended state. This is typically, although not necessarily, the position of the needle-housing assembly after assembly of the needle-housing assembly is complete and during transport of any device comprising the needle-housing assembly. Figure 9 shows an intermediate position in which the needle-housing assembly is partly extended. In this intermediate position, the needle has extended through the septum 70 of the distal shield 60, but has not extended through the septum 50 of the proximal shield 40. This is because the body 20 has not yet moved relative to the proximal shield by this point, but has moved in the distal direction relative to the distal shield. Figures 10 and 11 show a final position in which the needle-shield assembly is in a compressed position. In this final position, the needle has extended through both the septum 50 of the proximal shield 40 and the septum 70 of the distal shield 60.

The mechanism behind this sequence will now be explained. Once the needle-housing assembly starts to be compressed by pushing the proximal shield and the distal shield towards each other (for example when an autoinjector is pressed against an injection site), the first surface 26 of the housing engages the surface 42 of the proximal shield (overcoming the resistance from any connection between the distal shield and the body, such as the recesses 29 and corresponding arms 72 of the distal shield). As a result, the proximal shield and the body move in the distal direction relative to the distal shield. As this happens, the second surface 28 of the housing engages the surface of the distal shield, with the result that the first surface 26 of the housing is moved out of alignment with the surface 42 of the proximal shield (intermediate position). Once the first surface 26 of the housing is offset from (moved out of alignment with) the surface 42 of the proximal shield in the longitudinal direction, the proximal shield can continue to move in the distal direction relative to the distal shield, and now also moves in the distal direction relative to the body to reach a final position.

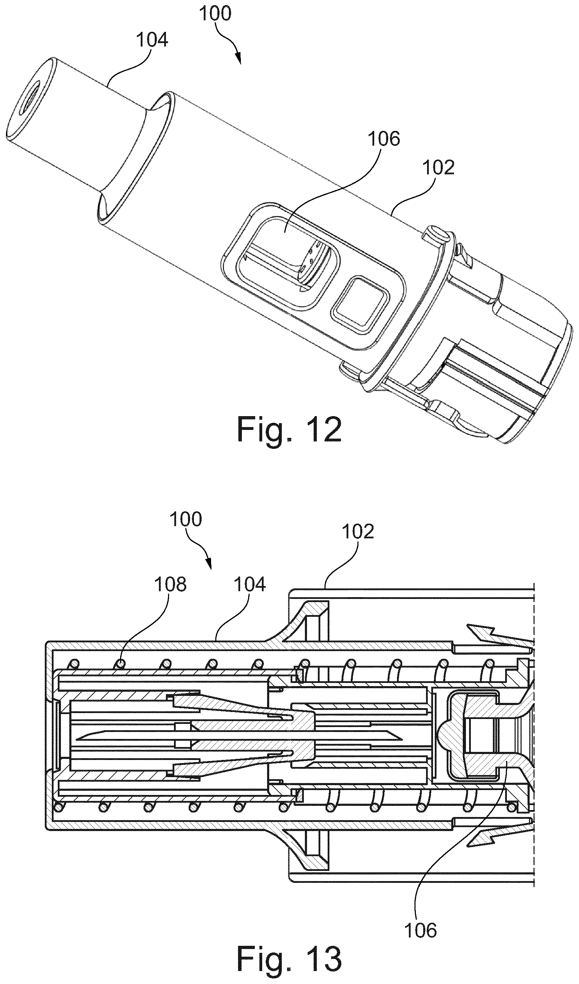

Figure 12 shows an example of a cassette 100 for an autoinjector which could incorporate the needle-housing assembly described above. The cassette 100 comprises a housing 102, a needle cover 104 and a medicament container 106. Figure 13 shows a cross-section of a portion of the cassette of Figure 12, showing in particular how the needle-housing assembly sits inside the needle cover and how the medicament container extends into an optional distal recess 79 of the needle-housing assembly (specifically a distal recess of the distal shield). A needle cover spring 108 is also visible. The housing 102, needle cover 104, medicament container 106 and needle cover spring 108 are shown as an example for context and are not intended to imply that these particular features must have these particular shapes, or that these features are even essential in such a device. More generally, the needle-housing assembly described herein could also be used in other injection devices, including pen injectors and autoinjectors such as single-use autoinjectors or multiple-use autoinjectors.

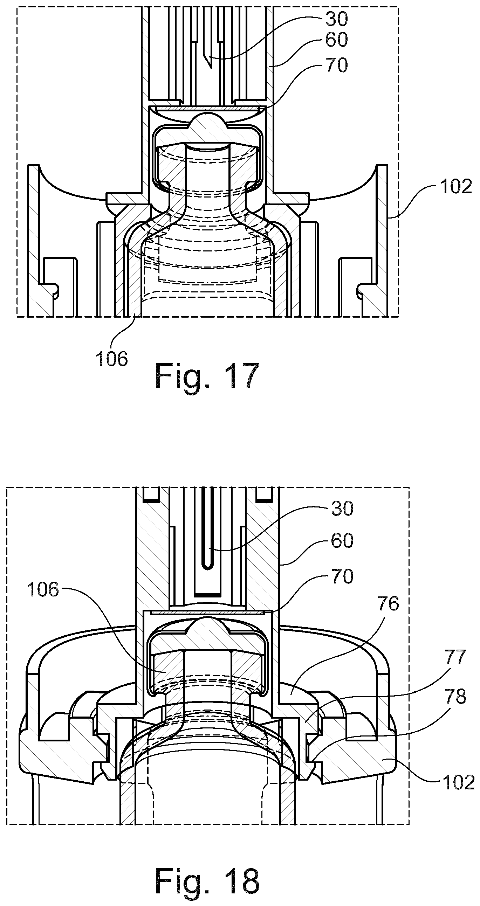

Figures 17 and 18 show one example of how the distal shield 60 could be attached to the cassette, in this case to the housing 102 (although distal shield 60 could alternatively be attached to another component such as a medicament container holder). The distal shield 60 is attached by a snap fit to the housing 102. In this case, this occurs by the protrusion 78 of the distal arm 77 of the distal shield 60 engaging a corresponding protrusion on the housing 102, although other snap fit structures or other attachment arrangements could be used instead. In this example, the distal shield is spaced apart from the medicament container. Alternatively, the distal shield could abut the medicament container.

The examples described above include a needle, and the medicament delivery member housing assembly is therefore described as a needle housing assembly. However, in general any medicament delivery member could be used; for example, a jet injector instead of a needle. In some cases, the medicament delivery member and/or the body comprises a piercing portion such as a spike, a ring or a needle to pierce the septums at the proximal end and/or at the distal end, particularly if one or both ends of the medicament delivery member is blunt.

In the examples above, the needle pierces the septum 70 of the distal shield first, and then subsequently pierces the septum 50 of the proximal shield. This means that in a complete device, where the proximal end is the end located closest to the medicament delivery site during device use, the needle first pierces the medicament cartridge and then extends out of the proximal end of the device. This is because the proximal shield first pushes the body and then disengages with the body once the distal shield engages the flexible arms of the body. However, the opposite orientation within a completed device is also possible, in which case the distal shield would actually be closer to the proximal end (i.e. the end closest to the medicament delivery site during device use) of the medicament delivery device than the proximal shield.

A needle housing with a particular shape is shown in the examples described above, but various modifications can be made to this shape. For example, the first surface 26 of the housing and the surface 42 of the proximal shield are shown as spaced apart in the longitudinal direction, but are alternatively not spaced apart in the longitudinal direction. The arms 24 could be extended further in the proximal direction (and the corresponding arms 48 of the proximal shield shortened or removed). The first surface 26 of the housing is shown at the proximal-most end of the flexible arm 24 of the housing, but could also be in a more distal position on the arm 24. The body 22 of the housing is shown as tubular, but could alternatively be less substantial; for example, the body could just be a ring.

The flexible arms 24 of the housing are shown being pushed inwards towards the axis so that the arms can pass into the distal shield in the examples given above. Alternatively, though, the arms 24 could be flexed so that they move in a different direction, for example circumferentially, to pass the restriction of the distal shield. In another alternative, the arms could be flexed outwards rather than inwards. The second surface 28 of the housing is shown as an integral part of the arm 24, but could alternatively be an extra feature such as a protrusion extending from the arm 24.

Two arms 24 are shown, but one, three or more arms could alternatively be provided. The same goes for many other features within the designs described herein, including the flanges 25 of the housing, the arms 72 of the distal shield and the distal arms 77 of the distal shield. Other features such as the ribs 45 of the proximal shield and the protrusions 65 of the distal shield can also be provided in quantities different to those shown in the examples.

The needle 30 is described above as a single needle. The needle could, however, comprise a proximal needle and a distal needle, with a channel through the body leading from the proximal needle to the distal needle. In the examples above, both ends of the needle have optional bevelled ends, which can help with penetration of the septums 50, 70.

Various alternatives are also possible for the shape of the proximal shield 40 and its constituent features. Typically, the proximal shield extends around a first end of the medicament delivery member. Providing arms 48 extending in the axial direction is optional, and the arms could extend in the radial direction instead. Alternatively, the use of arms could be avoided altogether by providing the surface 42 on a different part of the proximal shield. In general, the surface 42 of the proximal shield faces towards the distal end of the medicament delivery member housing assembly to interact with the first surface of the housing during use. The outer tubular portion 47 could instead be conical.

An optional extra feature is visible in Figure 4, where a distal portion 52 of the arm 48 of the proximal shield extends further in the distal direction than the surface 42, allowing a surface of the distal portion 52 (second surface of the proximal shield 40) to interact with (for example to abut) a corresponding surface (third surface 36) of the housing 20. This can help keep the surface 42 of the proximal shield aligned with the first surface 26 of the housing 20. The third surface 36 of the housing 20 and the surface of the distal portion face each other in a different direction to the surface 42 of the proximal shield and the first surface 26 of the housing 20. In the example shown in Figure 4, the third surface 36 of the housing 20 and the surface of the distal portion face each other in the radial direction.

The sealed proximal cavity 44 could just be between the housing and the proximal shield (rather than being formed by the housing, the proximal shield and the distal shield as described above), though this would need a different structure for the housing arms (e.g. with webbing between the arms). In general, providing sealed cavities for the medicament delivery member are not essential from a technical point of view (the medicament delivery member can still be protected without sealing), but are typically included to provide the added benefit of full sterility around the needle.

The septum 50 of the proximal shield is optional, and it may instead be an integral part of the proximal shield which is penetrated. The same goes for the septum 70 of the distal shield. The septums 50, 70 may be softer than the parts of the proximal shield and the distal shield respectively that they are attached to. One or both septums could be at the extreme end of the shield (as is the case for the septum 50 in Figure 3, which is at the proximal end of the proximal shield) or one or both septums could be set back somewhat (as is the case for the septum 70 in Figure 3, which is in a distal recess 79 formed by the distal end of the distal shield)
As with the proximal shield, various alternatives are possible for the shape of the distal shield 60, and some examples will now be given. The distal shield typically extends around a distal end of the medicament delivery member. In general, the surface 62 of the distal shield faces in the radial direction to interact with the second surface of the housing during use. The inner tubular portion 68 is optional; instead of being attached to the inner tubular portion 68, the arms 72 could be attached to other parts of the distal shield 60, such as to the outer tubular portion 67.

The flange 76 of the distal shield is optional. The flange 76 can be beneficial to limit the distal traverse available to the proximal shield (this could alternatively or additionally be limited by the proximal wall of the proximal shield and/or by another needle-housing component or by another cassette component) and/or to attach the needle-housing assembly within a medicament delivery device.

Provision of a seal 80 is optional, but is typically beneficial to help maintain sterility inside the needle-housing assembly. In general, a number of possible solutions are available to seal the gap between the proximal shield 40 and the distal shield 60. A number of possible solutions are also available to attach the proximal shield and the distal shield together, some of which include the seal 80. A number of examples are outlined below. In all these examples, the proximal shield and distal shield could be switched around.

For example, one approach simply uses a tight fit between the proximal shield and the distal shield, resulting in the proximal shield and the distal shield being held together by friction. Including an optional seal 80 in such an embodiment can help increase the friction. A second approach uses a snap fit, for example as shown in Figure 14. Again, a seal can optionally be included.

A third approach uses the seal 80 itself to hold the proximal shield and the distal shield together, by friction and/or with help from the structure of the proximal shield and/or the distal shield. For example, by attaching the seal 80 to one of the proximal shield and the distal shield, providing a recess 49 in the other of the proximal shield and the distal shield, and having the seal extend into the recess to restrict longitudinal movement of the proximal shield relative to the distal shield. The seal is typically flexible and can be pushed out of the way during use of the needle-housing assembly, allowing the proximal shield to move in the distal direction relative to the distal shield, although one or both of the proximal shield and the distal shield could instead be flexible. This approach can be seen in Figure 11. The seal could be fixed to the proximal housing instead of the distal housing, with the recess in the distal housing instead. The recess could be in a protrusion (such as the protrusion 84) so that the seal 80 is spaced apart from the shield that it is not attached to once it has disengaged from the recess during use, which can reduce friction from the seal during use. Having the seal 80 spaced apart in this manner can also provide a gap for gas in the needle-housing assembly to easily escape during use as the volume inside the needle-housing assembly reduces, which can also decrease resistance during use.

Several specific examples of combinations of approaches are shown herein. The first is shown in Figures 3 and 4 in particular, and comprises a seal 80 attached to the proximal shield 40. In this example, the outer tubular portion 47 of the proximal shield is spaced apart in the radial direction from the outer tubular portion 67 of the distal shield, with the seal 80 bridging the gap. Optional protrusions 65 (see Figure 7) on the proximal end of the distal shield (specifically on the outer tubular portion of the distal shield in this example) can help stop the proximal shield and the distal shield from coming apart by restricting the seal 80 from movement in the proximal direction relative to the distal shield. An optional snap fit is provided by ribs 45 on the proximal shield and corresponding protrusions 65 on the distal shield.

Another combination of approaches is shown in Figures 14 to 16. In this example, a snap fit is used, comprising interlocking protrusions 82, 83 on the proximal and distal shields respectively. In addition, a protrusion 84 on the distal shield 60 abuts the seal 80. When the proximal shield, and by extension the seal 80, move in the distal direction relative to the distal shield during use, the seal 80 is spaced apart from the distal shield, as can be seen in Figure 16. This can avoid friction from the seal during use. Having the seal 80 spaced apart in this manner can also provide a gap for gas in the needle-housing assembly to easily escape during use as the volume inside the needle-housing assembly reduces, which can also decrease resistance during use.

The example shown in Figures 12 and 13 is of a cartridge-based design, which can provide a single-use (or multiple-use) cassette for use with a (single-use or) multiple-use powerpack by providing a cassette and a powerpack as two separate parts that can be put together before use and taken apart after use so that the cassette can be discarded (or at least the needle-housing assembly can be discarded and replaced for reuse of the cassette) and the powerpack can be reused (or discarded separately). However, the needle-housing assembly could also be used in other types of autoinjector, such as autoinjectors that come as a functional device in a single piece, such as in the device described in WO2011/123024.

## Claims

1. A medicament delivery member housing assembly (10) extending along an axis (12) in a longitudinal direction (13) from a proximal end (14) to a distal end (16), wherein the proximal end of the medicament delivery member housing assembly is the end of the medicament delivery member housing assembly closest to the site of delivery of a medicament during use, the medicament delivery member housing assembly (10) comprising:
a housing (20) comprising a body (22) and a flexible arm (24), the flexible arm (24) extending in the longitudinal direction (13) from a proximal end moveable relative to the body (22) to a distal end attached to the body (22), wherein the flexible arm (24) comprises a first surface (26) facing towards the proximal end of the medicament delivery member housing assembly (10) and a second surface (28) facing in a radial direction (18) relative to the axis (12);
a medicament delivery member (30);
a proximal shield (40) extending around a first end of the medicament delivery member (30), the proximal shield (40) comprising a surface (42) facing towards the distal end of the medicament delivery member housing assembly (10) to interact with the first surface (26) of the housing (20) during use; and
a distal shield (60) extending around a distal end of the medicament delivery member (30), the distal shield (60) being slidable in the longitudinal direction (13) relative to the proximal shield (40), the distal shield (60) comprising a surface (62) facing in the radial direction (18) to interact with the second surface (28) of the housing (20) during use;
wherein the surface (62) facing in the radial direction is configured to interact with the second surface (28) of the housing (20) during use so as to move the first surface (26) out of alignment with the surface (42) facing towards the distal end of the medicament delivery member housing assembly (10).

2. The medicament delivery member housing assembly of claim 1, wherein the medicament delivery member is a needle attached to the housing.

3. The medicament delivery member housing assembly of claim 1 or 2, wherein the medicament delivery member housing assembly is configured to move from an initial extended position in which the medicament delivery member is fully enclosed by the medicament delivery member housing assembly to a final compressed position where the medicament delivery member extends out of the proximal end of the proximal shield and where the medicament delivery member extends out of the distal end of the distal shield.

4. The medicament delivery member housing assembly of any one of claims 1 to 3, wherein the medicament delivery member housing assembly is configured to move from the initial extended position to an intermediate partially-extended position and then to a final compressed position, wherein in the initial extended position, the first surface of the flexible arm and the surface of the proximal shield are aligned in the longitudinal direction,
wherein in the intermediate partially-extended position, the first surface of the flexible arm and the surface of the proximal shield are offset from one another in the longitudinal direction and adjacent to one another in the radial direction, and the second surface of the flexible arm is adjacent to the surface of the distal shield,
wherein in the final compressed position, the surface of the proximal shield is further from the proximal end of the proximal shield than the first surface of the flexible arm is from the proximal end of the proximal shield.

5. The medicament delivery member housing assembly of any of claims 1 to 4, wherein the second surface of the flexible arm faces away from the axis in the radial direction and the surface of the distal shield faces towards the axis in the radial direction.

6. The medicament delivery member housing assembly of any previous claim, wherein the proximal end of the proximal shield comprises a septum which the medicament delivery member can penetrate, and wherein the distal end of the distal shield comprises a septum which the medicament delivery member can penetrate.

7. The medicament delivery member housing assembly of any previous claim, wherein the first surface of the flexible arm and the surface of the proximal shield are spaced apart in the longitudinal direction.

8. The medicament delivery member housing assembly of any previous claim, comprising a seal arranged between the proximal shield and the distal shield.

9. The medicament delivery member housing assembly of claim 8, wherein the seal is attached to only one of the proximal shield and the distal shield.

10. The medicament delivery member housing assembly of claim 9, wherein the one of the proximal shield and the distal shield that the seal is not attached to comprises a recess, and the seal extends into the recess.

11. The medicament delivery member housing assembly of claim 9, wherein the one of the proximal shield and the distal shield that the seal is not attached to comprises a protrusion extending in the radial direction, and the seal abuts the protrusion.

12. The medicament delivery member housing assembly of any previous claim, wherein the housing is attached to the distal shield by a snap fit and/or the distal shield is attached to the proximal shield by a snap fit.

13. The medicament delivery member housing assembly of any previous claim, wherein the distal shield has a greater radius measured perpendicular to the axis than the proximal shield.

14. A cassette comprising a medicament delivery member housing assembly as described in any previous claim.

15. An autoinjector comprising a medicament delivery member housing assembly as claimed in any of claims 1 to 13 or comprising a cassette as claimed in claim 14.

## Patentansprüche

1. Medikamentenabgabeelementgehäuseanordnung (10), die sich entlang einer Achse (12) in einer Längsrichtung (13) von einem proximalen Ende (14) zu einem distalen Ende (16) erstreckt, wobei das proximale Ende der Medikamentenabgabeelementgehäuseanordnung das Ende der Medikamentenabgabeelementgehäuseanordnung ist, das während einer Verwendung der Abgabestelle eines Medikaments am nächsten liegt, die Medikamentenabgabeelementgehäuseanordnung (10) umfassend:
ein Gehäuse (20), umfassend einen Körper (22) und einen flexiblen Arm (24), wobei sich der flexible Arm (24) in der Längsrichtung (13) von einem proximalen Ende, das relativ zu dem Körper (22) bewegbar ist, zu einem distalen Ende, das an dem Körper (22) befestigt ist, erstreckt, wobei der flexible Arm (24) eine erste Oberfläche (26), die dem proximalen Ende der Medikamentenabgabeelementgehäuseanordnung (10) zugewandt ist, und eine zweite Oberfläche (28), die in eine radiale Richtung (18) relativ zu der Achse (12) gewandt ist, umfasst;
ein Medikamentenabgabeelement (30);
einen proximalen Schild (40), der sich um ein erstes Ende des Medikamentenabgabeelements (30) erstreckt, der proximale Schild (40) umfassend eine Oberfläche (42), die dem distalen Ende der Medikamentenabgabeelementgehäuseanordnung (10) zugewandt ist, um während der Verwendung mit der ersten Oberfläche (26) des Gehäuses (20) zusammenzuwirken; und
einen distalen Schild (60), der sich um ein distales Ende des Medikamentenabgabeelements (30) erstreckt, wobei der distale Schild (60) in der Längsrichtung (13) relativ zu dem proximalen Schild (40) verschiebbar ist, der distale Schild (60) umfassend eine Oberfläche (62), die in die radiale Richtung (18) gewandt ist, um während der Verwendung mit der zweiten Oberfläche (28) des Gehäuses (20) zusammenzuwirken; wobei die Oberfläche (62), die in die radiale Richtung gewandt ist, konfiguriert ist, um während der Verwendung mit der zweiten Oberfläche (28) des Gehäuses (20) zusammenzuwirken, um die erste Oberfläche (26) aus einer Ausrichtung mit der Oberfläche (42), die dem distalen Ende der Medikamentenabgabeelementgehäuseanordnung (10) zugewandt ist, zu bewegen.

2. Medikamentenabgabeelementgehäuseanordnung nach Anspruch 1, wobei das Medikamentenabgabeelement eine Nadel ist, die an dem Gehäuse befestigt ist.

3. Medikamentenabgabeelementgehäuseanordnung nach Anspruch 1 oder 2, wobei die Medikamentenabgabeelementgehäuseanordnung konfiguriert ist, um sich von einer anfänglichen ausgestreckten Position, in der das Medikamentenabgabeelement vollständig von der Medikamentenabgabeelementgehäuseanordnung umschlossen ist, zu einer endgültigen komprimierten Position zu bewegen, in der sich das Medikamentenabgabeelement aus dem proximalen Ende des proximalen Schildes heraus erstreckt und in der sich das Medikamentenabgabeelement aus dem distalen Ende des distalen Schildes heraus erstreckt.

4. Medikamentenabgabeelementgehäuseanordnung nach einem der Ansprüche 1 bis 3, wobei die Medikamentenabgabeelementgehäuseanordnung konfiguriert ist, um sich von der anfänglichen ausgestreckten Position zu einer zwischenliegenden teilweise ausgestreckten Position und dann zu einer endgültigen komprimierten Position zu bewegen,
wobei in der anfänglichen ausgestreckten Position die erste Oberfläche des flexiblen Arms und die Oberfläche des proximalen Schildes in der Längsrichtung ausgerichtet sind,
wobei in der zwischenliegenden teilweise ausgestreckten Position die erste Oberfläche des flexiblen Arms und die Oberfläche des proximalen Schildes in der Längsrichtung voneinander versetzt und in der radialen Richtung zueinander benachbart sind, und die zweite Oberfläche des flexiblen Arms zu der Oberfläche des distalen Schildes benachbart ist,
wobei in der endgültigen komprimierten Position die Oberfläche des proximalen Schildes weiter von dem proximalen Ende des proximalen Schildes als die erste Oberfläche des flexiblen Arms von dem proximalen Ende des proximalen Schildes entfernt ist.

5. Medikamentenabgabeelementgehäuseanordnung nach einem der Ansprüche 1 bis 4, wobei die zweite Oberfläche des flexiblen Arms in der radialen Richtung von der Achse abgewandt ist und die Oberfläche des distalen Schildes in der radialen Richtung der Achse zugewandt ist.

6. Medikamentenabgabeelementgehäuseanordnung nach einem der vorstehenden Ansprüche, wobei das proximale Ende des proximalen Schildes ein Septum umfasst, das das Medikamentenabgabeelement durchdringen kann, und wobei das distale Ende des distalen Schildes ein Septum umfasst, das das Medikamentenabgabeelement durchdringen kann.

7. Medikamentenabgabeelementgehäuseanordnung nach einem der vorstehenden Ansprüche, wobei die erste Oberfläche des flexiblen Arms und die Oberfläche des proximalen Schildes in der Längsrichtung beabstandet sind.

8. Medikamentenabgabeelementgehäuseanordnung nach einem der vorstehenden Ansprüche, umfassend eine Dichtung, die zwischen dem proximalen Schild und dem distalen Schild eingerichtet ist.

9. Medikamentenabgabeelementgehäuseanordnung nach Anspruch 8, wobei die Dichtung nur an einem von dem proximalen Schild und dem distalen Schild befestigt ist.

10. Medikamentenabgabeelementgehäuseanordnung nach Anspruch 9, wobei der eine von dem proximalen Schild und dem distalen Schild, an dem die Dichtung nicht befestigt ist, eine Vertiefung umfasst, und sich die Dichtung in die Vertiefung erstreckt.

11. Medikamentenabgabeelementgehäuseanordnung nach Anspruch 9, wobei der eine von dem proximalen Schild und dem distalen Schild, an dem die Dichtung nicht befestigt ist, einen Vorsprung umfasst, der sich in der radialen Richtung erstreckt, und die Dichtung an dem Vorsprung anliegt.

12. Medikamentenabgabeelementgehäuseanordnung nach einem der vorstehenden Ansprüche, wobei das Gehäuse an dem distalen Schild durch eine Schnappverbindung befestigt ist und/oder der distale Schild an dem proximalen Schild durch eine Schnappverbindung befestigt ist.

13. Medikamentenabgabeelementgehäuseanordnung nach einem der vorstehenden Ansprüche, wobei der distale Schild einen größeren Radius, der senkrecht zu der Achse gemessen wird, als der proximale Schild aufweist.

14. Kassette, umfassend eine Medikamentenabgabeelementgehäuseanordnung wie in einem der vorstehenden Ansprüche beschrieben.

15. Autoinjektor, umfassend eine Medikamentenabgabeelementgehäuseanordnung nach einem der Ansprüche 1 bis 13 oder umfassend eine Kassette nach Anspruch 14.

## Revendications

1. Ensemble logement d'élément d'administration de médicament (10) s'étendant le long d'un axe (12) dans une direction longitudinale (13) d'une extrémité proximale (14) à une extrémité distale (16), dans lequel l'extrémité proximale de l'ensemble logement d'élément d'administration de médicament est l'extrémité de l'ensemble logement d'élément d'administration de médicament la plus proche du site d'administration d'un médicament pendant l'utilisation, l'ensemble logement d'élément d'administration de médicament (10) comprenant :
un logement (20) comprenant un corps (22) et un bras flexible (24), le bras flexible (24) s'étendant dans la direction longitudinale (13) d'une extrémité proximale mobile par rapport au corps (22) à une extrémité distale fixée au corps (22), dans lequel le bras flexible (24) comprend une première surface (26) orientée vers l'extrémité proximale de l'ensemble logement d'élément d'administration de médicament (10) et une seconde surface (28) orientée dans une direction radiale (18) par rapport à l'axe (12) ;
un élément d'administration de médicament (30) ;
un écran proximal (40) s'étendant autour d'une première extrémité de l'élément d'administration de médicament (30), l'écran proximal (40) comprenant une surface (42) orientée vers l'extrémité distale de l'ensemble logement d'élément d'administration de médicament (10) pour interagir avec la première surface (26) du logement (20) pendant l'utilisation ; et
un écran distal (60) s'étendant autour d'une extrémité distale de l'élément d'administration de médicament (30), l'écran distal (60) pouvant coulisser dans la direction longitudinale (13) par rapport à l'écran proximal (40), l'écran distal (60) comprenant une surface (62) orientée dans la direction radiale (18) pour interagir avec la seconde surface (28) du logement (20) pendant l'utilisation ; dans lequel la surface (62) orientée dans la direction radiale est conçue pour interagir avec la seconde surface (28) du logement (20) pendant l'utilisation de manière à déplacer la première surface (26) hors de l'alignement avec la surface (42) orientée vers l'extrémité distale de l'ensemble logement d'élément d'administration de médicament (10).

2. Ensemble logement d'élément d'administration de médicament selon la revendication 1, dans lequel l'élément d'administration de médicament est une aiguille fixée au logement.

3. Ensemble logement d'élément d'administration de médicament selon la revendication 1 ou 2, dans lequel l'ensemble logement d'élément d'administration de médicament est conçu pour se déplacer d'une position étendue initiale dans laquelle l'élément d'administration de médicament est complètement enfermé par l'ensemble logement d'élément d'administration de médicament, à une position comprimée finale où l'élément d'administration de médicament s'étend hors de l'extrémité proximale de l'écran proximal et où l'élément d'administration de médicament s'étend hors de l'extrémité distale de l'écran distal.

4. Ensemble logement d'élément d'administration de médicament selon l'une quelconque des revendications 1 à 3, dans lequel l'ensemble logement d'élément d'administration de médicament est conçu pour se déplacer de la position étendue initiale à une position intermédiaire partiellement étendue, puis à une position comprimée finale,
dans lequel, dans la position initiale étendue, la première surface du bras flexible et la surface de l'écran proximal sont alignées dans la direction longitudinale,
dans lequel, dans la position intermédiaire partiellement étendue, la première surface du bras flexible et la surface de l'écran proximal sont décalées l'une par rapport à l'autre dans la direction longitudinale et adjacentes l'une à l'autre dans la direction radiale, et la seconde surface du bras flexible est adjacente à la surface de l'écran distal, dans lequel,
dans la position comprimée finale, la surface de l'écran proximal est plus éloignée de l'extrémité proximale de l'écran proximal que la première surface du bras flexible ne l'est de l'extrémité proximale de l'écran proximal.

5. Ensemble logement d'élément d'administration de médicament selon l'une quelconque des revendications 1 à 4, dans lequel la seconde surface du bras flexible est orientée à l'opposé de l'axe dans la direction radiale et la surface de l'écran distal est orientée vers l'axe dans la direction radiale.

6. Ensemble logement d'élément d'administration de médicament selon l'une quelconque revendication précédente, dans lequel l'extrémité proximale de l'écran proximal comprend un septum dans lequel l'élément d'administration de médicament peut pénétrer, et dans lequel l'extrémité distale de l'écran distal comprend un septum dans lequel l'élément d'administration de médicament peut pénétrer.

7. Ensemble logement d'élément d'administration de médicament selon l'une quelconque revendication précédente, dans lequel la première surface du bras flexible et la surface de l'écran proximal sont espacées dans la direction longitudinale.

8. Ensemble logement d'élément d'administration de médicament selon l'une quelconque revendication précédente, comprenant un joint agencé entre l'écran proximal et l'écran distal.

9. Ensemble logement d'élément d'administration de médicament selon la revendication 8, dans lequel le joint est attaché à un seul écran parmi l'écran proximal et l'écran distal.

10. Ensemble logement d'élément d'administration de médicament selon la revendication 9, dans lequel l'écran parmi l'écran proximal et l'écran distal auquel le joint d'étanchéité n'est pas fixé comprend un évidement, et le joint d'étanchéité s'étend dans l'évidement.

11. Ensemble logement d'élément d'administration de médicament selon la revendication 9, dans lequel l'écran parmi l'écran proximal et l'écran distal auquel le joint d'étanchéité n'est pas fixé comprend une saillie s'étendant dans la direction radiale, et le joint vient en butée contre la saillie.

12. Ensemble logement d'élément d'administration de médicament selon l'une quelconque revendication précédente, dans lequel le logement est fixé à l'écran distal par un encliquetage et/ou l'écran distal est fixé à l'écran proximal par un encliquetage.

13. Ensemble logement d'élément d'administration de médicament selon l'une quelconque revendication précédente, dans lequel l'écran distal a un rayon plus grand mesuré perpendiculairement à l'axe que l'écran proximal.

14. Cassette comprenant un ensemble logement d'élément d'administration de médicament tel que décrit dans l'une quelconque revendication précédente.

15. Auto-injecteur comprenant un ensemble logement d'élément d'administration de médicament selon l'une quelconque des revendications 1 à 13 ou comprenant une cassette selon la revendication 14.
